(19) Europäisches Patentamt
European Patent Office
Office européen des brevets

(11) Publication number: **0 130 818 B1**

(12) **EUROPEAN PATENT SPECIFICATION**

(45) Date of publication of patent specification: **08.04.87**

(51) Int. Cl.4: **C 07 C 143/12,** C 11 D 1/28

(21) Application number: **84304478.5**

(22) Date of filing: **29.06.84**

(54) **Process for the handling of dialkyl sulphosuccinates.**

(30) Priority: **01.07.83 GB 8317883**

(43) Date of publication of application:
**09.01.85 Bulletin 85/02**

(45) Publication of the grant of the patent:
**08.04.87 Bulletin 87/15**

(84) Designated Contracting States:
**AT BE CH DE FR IT LI NL SE**

(56) References cited:
**DE-A-1 912 531**
**DE-A-2 110 030**
**DE-A-2 546 847**
**CHEMICAL ABSTRACTS, vol. 96, No. 10, 8th March 1982, Columbus, Ohio, USA; T. SUZAWA et al. "Viscometric studies on the interaction of poly(vinyl alcohol) with sodium 2-sulfonatofatty acid methyl esters", page 27, column 2, abstract no. 69773d**

(73) Proprietor: **UNILEVER NV**
**Burgemeester s'Jacobplein 1 P.O. Box 760**
**NL-3000 DK Rotterdam (NL)**

(72) Inventor: **Rand, John Arthur**
**Ridder van Catsweg 32**
**NL-2805 BA Gouda (NL)**
Inventor: **Stanley, Christopher Paul**
**7 High Ash Mount Alwoodley**
**Leeds 17 West Yorkshire (GB)**

(74) Representative: **Mole, Peter Geoffrey et al**
**UNILEVER PLC Patent Division P.O. Box 68**
**Unilever House Blackfriars**
**London EC4P 4BQ (GB)**

(56) References cited:
**CHEMICAL ABSTRACTS, vol. 83, no. 14, 6th October 1975, Columbus, Ohio, USA; Z MORI "Surface-treating compositions", page 209, column 2, abstract no. 117629s**

**EP 0 130 818 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to the preparation of salts of dialkyl esters of sulphosuccinic acid, hereinafter referred to as dialkyl sulphosuccinates. These materials are useful *inter alia* as detergent-active agents.

Dialkyl sulphosuccinates are materials of the general formula I.

$$\begin{array}{ll} CH_2 \text{——} & CH\text{—}SO_3X \\ | & | \\ COOR_1 & COOR_2 \end{array}$$

wherein $R_1$ and $R_2$ are straight-chain or branched-chain alkyl groups, and may be the same or different, and X is a monovalent cation or 1/m of an *m*-valent cation. For detergent activity, the alkyl groups $R_1$ and $R_2$, which may be the same or different, generally have 4 to 12 carbon atoms, and X is a solubilising cation, for example, alkali metal, ammonium, substituted ammonium or magnesium.

The synthesis of dialkyl sulphosuccinates is well documented in the literature, for example in US 2 028 091 and GB 760 121 (American Cyanamid), DD 75075 (Weigner), GB 1 576 019 (BASF), GB 1 215 561 (Melle Bezons), and GB 1 527 020 (Chemische Fabrik Pfersee). The preferred method for making these materials is by bisulphite addition to the corresponding dialkyl maleates and/or fumarates. The reaction is generally carried out in an aqueous medium, optionally in the presence of ethanol, at elevated temperatures above 85°C. The sulphiting agent is generally a water-soluble sulphite-ion-generating salt, for example, an alkali metal or ammonium sulphite, bisulphite or metabisulphite.

The present invention is especially concerned with the preparation of dialkyl sulphosuccinates for use in liquid detergent compositions. For this end-use, the reaction may conveniently be carried out in relatively large quantities of water, to give a final aqueous product having a concentration of about 30 to 50% by weight. If desired, small amounts of ethanol may also be present. After the reaction is complete the mixture is cooled from the relatively high reaction temperature above 85°C to a desired temperature for further processing or storage.

The product mixture is a non-Newtonian pseudo-plastic liquid and during this cooling stage an unexpected problem has been encountered in that as the temperature falls the viscosity rises sharply so that the reaction mixture becomes a highly viscous mass which is very difficult to handle. The highly viscous mass cannot readily be cooled further using conventional cooling equipment because of poor heat transfer and poor fluid flow properties. The tendency to form a highly viscous mass during cooling is especially marked when dialkyl sulphosuccinates of more than one alkyl chain length are present.

Various measures have been tried in an attempt to alleviate this problem. Varying the electrolyte content has been found to be ineffective, as has the addition of other detergent-active materials, for example, alkyl ether sulphates.

The problem can be circumvented by working at lower concentrations or by using large amounts of ethanol, but the final product is then no longer tailored to end-user requirements. Large amounts of ethanol give products of unacceptably low viscosity.

It has now been found, according to the present invention, that in the presence of relatively low amounts of ethanol the addition of urea in a particular amount at or near the beginning of the cooling stage is highly effective in preventing a steep rise in viscosity. This is surprising because above about 75°C the decomposition of urea into ammonia and carbon dioxide starts to become significant, yet in the process of the invention it is generally necessary to add it to the reaction mixture at temperatures not significantly below this figure.

The presence of urea in the final product at the sort of levels in question is also advantageous because, as is well known in the art, urea is a useful hydrotrope and formulation aid in liquid detergent compositions.

The present invention accordingly provides a process for the preparation of an aqueous composition containing from 30 to 50% by weight of at least one salt of a $C_4$—$C_{16}$ dialkyl ester of sulphosuccinic acid, which comprises the steps of:

(a) chemically synthesising one or more $C_4$—$C_{16}$ dialkyl esters of sulphosuccinic acid in an aqueous reaction mixture at a concentration of from 30 to 50% by weight at a temperature above 85°C,

(b) cooling the mixture, in the presence of from 2 to 20% by weight of ethanol, to a temperature below 75°C, preferably below 70°C, at which the mixture is still fluid,

(c) adding to the cooled reaction mixture from 10 to 30% by weight of urea, based on the dialkyl sulphosuccinate; and

(d) cooling the mixture further to a desired temperature for storage or further processing.

Preferably step (b) comprises cooling the mixture to a temperature at which its viscosity does not exceed 50 poise, and more preferably does not exceed 20 poise, at a shear rate of 110 $s^{-1}$.

The dialkyl sulphosuccinates with which the invention is concerned preferably have alkyl chain lengths within the range of from $C_4$ to $C_{10}$, more preferably $C_6$ to $C_8$. Dialkyl sulphosuccinates of this chain length are especially suitable for use in high foaming light duty liquid products.

If desired, mixtures of dialkyl sulphosuccinates of different chain lengths may be prepared by the process of the invention. In such mixtures, the individual dialkyl sulphosuccinates may be either symmetrical (both alkyl groups the same) or unsymmetrical (with two different alkyl groups).

Of especial interest are mixed dialkyl sulphosuccinates derived from mixtures of two or more alcohols, notably the mixtures described and claimed in GB 2 108 520 (Unilever) and British Patent Application No. 84 01449 (Unilever). An example of the former type of product is the $C_6/C_8$ mixed system derived from a mixture of $C_6$ and $C_8$ alcohols and containing the symmetrical dihexyl and dioctyl sulphosuccinates as well as the unsymmetrical hexyl octyl sulphosuccinate. The latter material is described and claimed in GB 2 105 325 (Unilever).

Mixed $C_7/C_8$ and $C_6/C_7/C_8$ systems derived from mixtures of the corresponding alcohols are described and claimed in the aforementioned British Patent Application No. 84 01449 and are also highly preferred in the context of the present invention.

The synthesis of the dialkyl sulphosuccinate in step (a) may be carried out by any method suitable for operation under aqueous conditions to give a final product having a concentration of 30 to 50% by weight, preferably 35 to 40% by weight, and which requires a reaction temperature above 85°C. As previously indicated, the normal and most convenient synthetic route is by way of bisulphite addition to the corresponding dialkyl maleate and/or fumarate. These in turn may be prepared by esterification of the corresponding alcohol or mixture of alcohols with maleic anhydride or maleic acid, preferably maleic anhydride. The sulphitation process is well known in the art and details are given, for example, in the patent specifications mentioned previously.

Thus according to a preferred embodiment of the invention step (a) comprises reacting together at least one $C_4$—$C_{16}$ dialkyl maleate and/or fumarate and a water-soluble sulphite-ion-generating material.

The sulphiting agent may be any material that yields sulphite ions in aqueous solution. Suitable materials include the alkali metal and ammonium sulphites, bisulphites and metabisulphites. Preferred materials are sodium metabisulphite and sodium sulphite.

As previously mentioned, the reaction is carried out in a relatively large volume of water, the concentration of the starting materials being chosen so that the final product has a concentration suitable for its intended end-use. In general the conversion of maleate/fumarate to sulphosuccinate is highly efficient (95—100%) and the yield of sulphosuccinate is of the order of 98% of the theoretical yield, so little or no correction for loss during the reaction is required in choosing the initial concentration.

The reaction is generally carried out under reflux conditions under ambient or elevated pressure. The reaction temperature will thus exceed 85°C, and will generally exceed 100°C. Typically the temperature will be in the range of from 85 to 130°C. At the end of the reaction the mixture will be at a relatively high temperature and may then be cooled. Cooling is clearly desirable in order to reach a convenient handling temperature, and is also important in order to minimise hydrolysis of the product.

It is during this stage of the process that the sudden increase in viscosity takes place that makes further cooling and handling so difficult. With the $C_6/C_8$ dialkyl sulphosuccinate mix the referred to earlier, the viscosity begins to rise steeply at temperatures around 70 to 80°C.

According to the present invention urea is added during the cooling stage, in time to prevent this dramatic viscosity increase. The addition of urea must not be made too soon, because of its tendency to start to decompose at a significant rate at temperatures above 75°C, but on the other hand it must be added before the viscosity has risen to a level at which further cooling and handling become difficult. The lowest temperature at which the urea can be added depends on the rheology of the particular material or materials present. For the $C_6/C_8$ mixture, this lower limit will generally be around 60°C, and the addition of urea should be carried out at a temperature in the 60 to 70°C range, preferably at about 65°C.

Once urea has been added, the rheology of the mixture changes completely, the viscosity typically falling by a factor of 10 and then increasing gradually as the temperature is reduced further.

The urea is added in an amount of from 10 to 30%, more preferably 15 to 25% by weight, based on the dialkyl sulphosuccinate present. At a 40% dialkyl sulphosuccinate level the optimum urea level of about 20% by weight is equivalent to about 8% by weight based on the total composition.

The urea is preferably added in the form of a solid, as powder or granules, conveniently in the form of prills.

It is essential that some lower alcohol, preferably ethanol, be present during the cooling stage. If alcohol has not been present during the manufacturing step (a), it may be introduced at the end of the reaction before cooling. At least 2% by weight of alcohol, preferably 3 to 6% by weight, should be present during cooling, the percentages being based on the total reaction mixture. Amounts of alcohol exceeding 20% by weight are undesirable for product formulation reasons.

The product, having a dialkyl sulphosuccinate level of 30 to 50% by weight, preferably 35 to 40% by weight, and a urea level preferably in the 6 to 10% by weight range, can conveniently be used for formulating a range of liquid detergent products.

The invention will now be illustrated by the following non-limiting Examples.

Example 1-

An aqueous composition containing about 39% by weight (in total) of the mixed $C_6$ and $C_8$ dialkyl sulphosuccinates described in GB 2 108 520 (Unilever), was prepared by reacting 24 parts $C_6/C_8$ dialkyl maleates with 8.7 parts of sodium metabisulphite in the presence of an emulsifying mix of 43.6 parts water, 3.2 parts industrial ethanol and 20 parts of a 40% $C_6/C_8$ dialkyl sulphosuccinate mixture (product from a previous batch).

The reaction was carried out at 90—95°C, in a stirred reactor fitted with a torque measuring device. After 5 hours, the reaction was essentially complete with final mixture containing 39.2% dialkyl sulphosuccinate and only 0.002% dialkyl maleate. At the end of the reaction, the net torque exerted by the stirrer on the liquid was about 1.0 Nm. On cooling to 62°C, the net torque rose to about 12 Nm indicating about a 10 fold increase in viscosity. 8 parts of urea were then added, and the net torque fell immediately to about 1.5 Nm. Further cooling was then readily accomplished, during which the net torque rose slowly to reach a maximum of about 9.7 Nm at 30°C before falling again rapidly as the temperature was further reduced.

The stirrer torque is related linearly to the viscosity, but the relationship is complex and it is not possible to deduce the actual viscosity figures from the torque values. These do, however, show how the viscosity changed on cooling and on the addition of urea.

Example 2

Samples of the dialkyl sulphosuccinate mixture prepared as in Example 1, at a concentration of 40% by weight and containing 3% by weight of ethanol, were heated to various temperatures as detailed below in the presence of various amounts of urea, and their viscosities at a shear rate of 110 $s^{-1}$ were measured by means of a Haake viscometer. The results were as shown in the Table below, the urea percentages being based on the dialkyl sulphosuccinate.

| Urea level (%) | Viscosity (poise) at temperature (°C) of | | | | | |
|---|---|---|---|---|---|---|
| | 60 | 50 | 45 | 40 | 35 | 30 |
| 0 | 40 | 35 | 28 | 18 | 9 | 3 |
| 7.5 | 23 | 30 | 22 | 15 | 10 | 7 |
| 15 | 1 | 31 | 27 | 21 | 20 | 21 |
| 20 | 0.6 | 0.9 | 4.5 | 26 | 28 | 1.5 |

It will be seen that with this particular dialkyl sulphosuccinate system and at this particular concentration level, a 7.5% urea level is clearly inadequate over the whole temperature range and a 15% urea level is unsatisfactory at the lower end of the range. The 20% level is good over the whole range, except for a narrow viscosity peak at 35 to 40°C.

**Claims**

1. A process for the preparation of an aqueous composition containing from 30 to 50% by weight of at least one salt of a $C_4$—$C_{16}$ dialkyl ester of sulphosuccinic acid, characterised in that it comprises the steps of:

(a) chemically synthesising one or more $C_4$—$C_{16}$ dialkyl esters of sulphosuccinic acid in an aqueous reaction mixture at a concentration of from 30 to 50% by weight at a temperature above 85°C,

(b) cooling the mixture, in the presence of from 2 to 20% by weight of a lower alcohol, to a temperature below 75°C at which the mixture is still fluid,

(c) adding to the cooled reaction mixture from 10 to 30% by weight of urea, based on the dialkyl sulphosuccinate; and

(d) cooling the mixture further to a desired temperature for storage or further processing.

2. A process according to claim 1, characterised in that step (b) comprises cooling the mixture to a temperature below 70°C at which the mixture is still fluid.

3. A process according to claim 1, characterised in that step (b) comprises cooling the mixture to a temperature at which the viscosity does not exceed 50 poise at a shear rate of 110 $s^{-1}$.

4. A process according to claim 3, characterised in that step (b) comprises cooling the mixture to a temperature at which the viscosity does not exceed 20 poise at a shear rate of 110 $s^{-1}$.

5. A process according to any one of claims 1 to 4, characterised in that the dialkyl sulphosuccinate has a chain length within the range of from $C_4$ to $C_{10}$.

6. A process according to claim 5, characterised in that the dialkyl sulphosuccinate has a chain length within the range of from $C_6$ to $C_8$.

7. A process according to any one of claims 1 to 6, characterised in that the dialkyl sulphosuccinate includes material of more than one chain length.

8. A process according to claim 7, characterised in that the dialkyl sulphosuccinate comprises a mixture of dioctyl, dihexyl and hexyl octyl sulphosuccinates.

9. A process according to any one of claims 1 to 8, characterised in that the reaction mixture at the end of step (a) has a dialkyl sulphosuccinate concentration of from 35 to 40% by weight.

10. A process according to any one of claims 1 to 9, characterised in that step (b) is carried out in the presence of from 3 to 6% by weight of the lower alcohol.

11. A process according to any one of claims 1 to 10, characterised in that the lower alcohol comprises ethanol.

12. A process according to any one of claims 1 to 11, characterised in that in step (c) the amount of urea added is within the range of from 15 to 25% by weight, based on the dialkyl sulphosuccinate.

13. A process according to any one of claims 1 to 12, characterised in that the urea is added at a temperature within the range of from 60 to 70°C.

14. A process according to any one of claims 1 to 13, characterised in that step (a) comprises reacting together at least one $C_4$—$C_{16}$ dialkyl maleate and/or fumarate and a water-soluble sulphite-ion-generating material.

**Patentansprüche**

1. Verfahren zur Herstellung einer wäßrigen Zusammensetzung enthaltend von 30 bis 50 Gew.-% mindestens eines Salzes eines $C_4$—$C_{16}$-Dialkylesters der Sulfobernsteinsäure, dadurch gekennzeichnet, daß es die Schritte:

(a) chemische Synthetisierung von einem oder mehreren $C_4$—$C_{16}$-Dialkylestern der Sulfobernsteinsäure in einer wäßrigen Reaktionsmischung bei einer Konzentration von 30 bis 50 Gew.-% bei einer Temperatur über 85°C,

(b) Kühlen der Mischung in Gegenwart von 2 bis 20 Gew.-% eines niederen Alkohols auf eine Temperatur unter 75°C, bei der die Mischung noch flüssig ist,

(c) Zugeben zu der gekühlten Reaktionsmischung von 10 bis 30 Gew.-% Harnstoff, bezogen auf das Dialkylsulfosuccinat und

(d) Kühlen der Mischung weiter auf eine gewünschte Temperatur zur Lagerung oder weiteren Bearbeitung,

umfaßt.

2. Verfahren nach Anspruch 1, dadurch gekennzeichnet, daß Schritt (b) das Kühlen der Mischung auf eine Temperatur unter 70°C, bei der die Mischung noch flüssig ist, umfaßt.

3. Verfahren nach Anspruch 1, dadurch gekennzeichnet, daß Schritt (b) das Kühlen der Mischung auf eine Temperatur, bei der die Viskosität 50 Poise bei einer Scherrate von 110 $s^{-1}$ nicht überschreitet, umfaßt.

4. Verfahren nach Anspruch 3, dadurch gekennzeichnet, daß Schritt (b) das Kühlen der Mischung auf eine Temperatur, bei der die Viskosität 20 Poise bei einer Scherrate von 100 $s^{-1}$ nicht überschreitet, umfaßt.

5. Verfahren nach einem der Ansprüche 1 bis 4, dadurch gekennzeichnet, daß das Dialkylsulfosuccinat eine Kettenlänge innerhalb des Bereiches von $C_4$ bis $C_{10}$ hat.

6. Verfahren nach Anspruch 5, dadurch gekennzeichnet, daß das Dialkylsulfosuccinat eine Kettenlänge innerhalb des Bereiches von $C_6$ bis $C_8$ hat.

7. Verfahren nach einem der Ansprüche 1 bis 6, dadurch gekennzeichnet, daß das Dialkylsulfosuccinat Material von mehr als einer Kettenlänge umfaßt.

8. Verfahren nach Anspruch 7, dadurch gekennzeichnet, daß das Dialkylsulfosuccinat eine Mischung von Dioctyl-, Dihexyl- und Hexyl-octyl-sulfosuccinaten umfaßt.

9. Verfahren nach einem der Ansprüche 1 bis 8, dadurch gekennzeichnet, daß die Reaktionsmischung am Ende von Schritt (a) eine Dialkylsulfosuccinat-Konzentration von 35 bis 40 Gew.-% hat.

10. Verfahren nach einem der Ansprüche 1 bis 9, dadurch gekennzeichnet, daß Schritt (b) in Gegenwart von 3 bis 6 Gew.-% des niederen Alkohols durchgeführt wird.

11. Verfahren nach einem der Ansprüche 1 bis 10, dadurch gekennzeichnet, daß der niedere Alkohol Ethanol umfaßt.

12. Verfahren nach einem der Ansprüche 1 bis 11, dadurch gekennzeichnet, daß in Schritt (c) die Menge des zugegebenen Harnstoffs innerhalb des Bereiches von 15 bis 25 Gew.-%, bezogen auf das Dialkylsulfosuccinat beträgt.

13. Verfahren nach einem der Ansprüche 1 bis 12, dadurch gekennzeichnet, daß der Harnstoff bei einer Temperatur innerhalb des Bereiches von 60 bis 70°C zugegeben wird.

14. Verfahren nach einem der Ansprüche 1 bis 13, dadurch gekennzeichnet, daß Schritt (a) das gemeinsame Umsetzen von mindestens einem $C_4$—$C_{16}$-Dialkylmaleat und/oder -Fumarat und einem wasserlöslichen, Sulfit-Ion-erzeugenden Material umfaßt.

**Revendications**

1. Procédé de préparation d'une composition aqueuse contenant de 30 à 50% en poids d'au moins un sel d'un dialcoyle-ester en $C_4$ à $C_{16}$ d'acide sulfosuccinique, caractérisé en ce qu'il comprend les étapes consistant à:

(a) synthétiser chimiquement un ou plusieurs dialcoyl-esters en $C_4$ à $C_{16}$ d'acide sulfosuccinique dans

un milieu réactionnel aqueux à une concentration de 30 à 50% en poids à une température supérieure à 85°C,

(b) refroidir le mélange, en présence de 2 à 20% en poids d'un alcool inférieur, à une température inférieure à 75°C à laquelle le mélange est encore fluide,

(c) ajouter au mélange réactionnel refroidi de 10 à 30% en poids d'urée, sur la base du dialcoyl -sulfosuccinate; et

(d) refroidir plus avant le mélange à une température désirée aux fins de conservation ou de traitement ultérieurs.

2. Procédé selon la revendication 1, caractérisé en ce que l'étape (b) comprend le refroidissement du mélange à une température inférieure à 70°C à laquelle le mélange est toujours fluide.

3. Procédé selon la revendication 1, caractérisé en ce que l'étape (b) comprend le refroidissement du mélange à une température à laquelle la viscosité ne dépasse pas 50 poises à un taux de cisaillement de 110 $s^{-1}$.

4. Procédé selon la revendication 3, caractérisé en ce que l'étape (b) comprend le refroidissement du mélange à une température à laquelle la viscosité ne dépasse pas 20 poises à un taux de cisaillement de 110 $s^{-1}$.

5. Procédé selon l'une quelconque des revendications 1 à 4, caractérisé en ce que le dialcoyl -sulfosuccinate a une longueur de chaîne située dans l'intervalle allant de $C_4$ à $C_{10}$.

6. Procédé selon la revendication 5, caractérisé en ce que le dialcoyl -sulfosuccinate à une longueur de chaîne située dans l'intervalle allant de $C_6$ à $C_8$.

7. Procédé selon l'une quelconque des revendications 1 à 6, caractérisé en ce que le dialcoyl -sulfosuccinate comprend une matière de plus d'une longueur de chaîne.

8. Procédé selon la revendication 7, caractérisé en ce que le dialcoyl -sulfosuccinate comprend un mélange de dioctyl-, dihexyl- et hexyl -octyl -sulfosuccinates.

9. Procédé selon l'une quelconque des revendications 1 à 8, caractérisé en ce que le mélange réactionnel à la fin de l'étape (a) a une concentration de dialcoyl -sulfosuccinate de 35 à 40% en poids.

10. Procédé selon l'une quelconque des revendications 1 à 9, caractérisé en ce que l'étape (b) est conduite en présence de 3 à 6% en poids de l'alcool inférieur.

11. Procédé selon l'une quelconque des revendications 1 à 10, caractérisé en ce que l'alcool inférieur comprend l'éthanol.

12. Procédé selon l'une quelconque des revendications 1 à 11, caractérisé en ce que dans l'étape (c) la quantité d'urée ajoutée est dans l'intervalle allant de 15 à 25% en poids, sur la base du dialcoyl -sulfosuccinate.

13. Procédé selon l'une quelconque des revendications 1 à 12, caractérise en ce que l'urée est ajoutée à une température située dans l'intervalle allant de 60 à 70°C.

14. Procédé selon l'une quelconque des revendications 1 à 13, caractérisé en ce que l'étape (a) comprend la réaction l'un avec l'autre d'au moins un dialcoyl-maléate et/ou -fumarate en $C_4$ à $C_{16}$ et d'une matière génératrice d'ions sulfites solubles dans l'eau.